(19)

**Europäisches Patentamt**
**European Patent Office**
**Office européen des brevets**

(11) **EP 4 494 632 A1**

(12) **EUROPEAN PATENT APPLICATION**

(43) Date of publication:
**22.01.2025 Bulletin 2025/04**

(21) Application number: **23382737.7**

(22) Date of filing: **19.07.2023**

(51) International Patent Classification (IPC):
*A61K 9/00* (2006.01)    *A61K 9/06* (2006.01)
*A61K 31/23* (2006.01)    *A61K 47/10* (2017.01)
*A61K 47/14* (2017.01)

(52) Cooperative Patent Classification (CPC):
**A61K 47/14; A61K 9/0043; A61K 9/006;**
**A61K 9/06; A61K 31/23; A61K 47/10**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicants:
• **Consejo Superior de Investigaciones Científicas**
  **(CSIC)**
  **28006 Madrid (ES)**
• **Universitat de Barcelona**
  **08028 Barcelona (ES)**
• **Universidad de Granada**
  **18071 Granada (ES)**

(72) Inventors:
• **Alonso Merino, Cristina**
  **Barcelona (ES)**

• **Martí Gelabert, Meritxell**
  **Barcelona (ES)**
• **Coderch Negra, Mª Luisa**
  **Barcelona (ES)**
• **Calpena Campmany, Ana Cristina**
  **Barcelona (ES)**
• **Mallandrich Miret, Mireia**
  **Barcelona (ES)**
• **Pérez García, Mª Luisa**
  **Barcelona (ES)**
• **Clares Naveros, Beatriz**
  **Granada (ES)**
• **Pérez González, Noelia**
  **Granada (ES)**

(74) Representative: **Pons**
**Glorieta Rubén Darío 4**
**28010 Madrid (ES)**

(54) **LIPOPHILIC-BASED COMPOSITION**

(57)    The present invention relates to a lipophilic-based composition and to the use thereof as an agent for the impermeabilization of oral or nasal mucosae, therefore, said composition also relates to the use thereof as a medical product, particularly for preventing viral infections, such as SARS-CoV-2, and to its use for preventing contaminants, such as biocides, from penetrating through the oral or nasal mucosae. Furthermore, the invention relates to a medical product comprising the composition of the invention and to a medical device thereof.

**EP 4 494 632 A1**

**Description**

The present invention relates to a lipophilic-based composition and to the use thereof as an agent for the impermeabilization of oral or nasal mucosae, therefore, said composition also relates to the use thereof as a medical product, particularly for preventing viral infections, such as SARS-CoV-2, and to its use for preventing contaminants, such as biocides, from penetrating through the oral or nasal mucosae. Furthermore, the invention relates to a medical product comprising the composition of the invention and to a medical device thereof. Therefore, the invention could be comprised in the field of pharmaceutical technology.

## STATE OF THE ART

The permeation of SARS-CoV-2 through the nasal or oral mucosa in contrast to its low penetration through the keratinized tissue of the stratum corneum of the skin is widely known. This is mainly due to the different lipid composition and packaging structures formed. It has been shown that, for the skin, the main determining factor of the barrier function is the lipid content of the epidermal stratum corneum, rather than the thickness or number of corneocyte layers present (S.H. White, D. Mirejovsky, G.I. King, Structure of Lamellar Lipid Domains and Corneocyte Envelopes of Murine Stratum Corneum. An X-ray Diffraction Study, Biochemistry 1988, 27, 3725-3732; Bouwstra J, Pilgram G, Gooris G, Koerten H, Ponec M. New aspects of the skin barrier organization. Skin Pharmacol Appl Skin Physiol. 2001; 14 Suppl 1, 52-62). In the stratum corneum of the skin, ceramides, fatty acids, and cholesterol are the main lipids that determine the permeability barrier (B. Janůšová, J. Zbytovská, P. Lorenc, H. Vavrysová, K. Palát, A. Hrabálek, K. Vávrová, Effect of ceramide acyl chain length on skin permeability and thermotropic phase behavior of model stratum corneum lipid membranes, Biochim. Biophys. *Acta - Mol. Cell Biol. Lipids.* **2011,** *1811, 129-137;* D. Kessner, A. Ruettinger, M.A. Kiselev, S. Wartewig, R.H.H. Neubert, Properties of ceramides and their impact on the stratum corneum structure: A review - Part 2: Stratum corneum lipid model systems, Skin Pharmacol. Physiol. 2008, 21, 58-74).

The oral mucosa consists of connective tissue known as lamina propria which is covered by a stratified squamous epithelium. The oral mucosa is covered by a stratified epithelium, the maturation pattern of which is very similar to that of the skin, which provides a barrier against the aggression of endogenous or exogenous substances present in the oral cavity and also prevents the loss of material from the underlying tissue. Morphological diversity ranging from regions of orthokeratinized mucosa to non-keratinized mucosa can be observed.

In general, it is accepted that the resistance of the oral/nasal mucosa to diffusion is mainly associated with the intercellular lipids of the outer layers of the tissue. The nature of the intercellular material is therefore an important determining factor in oral epithelial permeability. In keratinized oral epithelia, the lipid components present are similar to those of the epidermis; the main components are neutral lipids which consist mainly of ceramides and acylceramides and are derived from the lamellae of membrane-coating granules. The epithelium of non-keratinized oral regions contains no acylceramides or acylglycosylceramides and small amounts of ceramide, but relatively high amounts of glycosylceramide. Ceramides are present only in small amounts in non-keratinized epithelia, there is no mechanism to convert glycosylceramide to ceramide, as occurs in keratinized epithelium. Other physiological characteristics that distinguish mucosal tissues from the skin, such as an extensive vasculature, their moist surface and the presence of mucus, should also be taken into account. Mucosal tissues are covered with negatively charged mucus which contains large glycoproteins called mucins. Mucus and saliva play an important role during penetration and can contribute to the barrier layer of mucosal tissues (Beste Kinikoglu, Odile Damour, Vasif Hasirci, Tissue engineering of oral mucosa: a shared concept with skin J Artif Organs 2015, 18, 8-19; Amir H Shojael. Buccal Mucosa as A Route For Systemic Drug Delivery: A Review. J. Pharm. Pharmaceut. Sci. 1988, 1 (1), 15-30; Winning TA, Townsend GC. Oral mucosal embryology and histology. Clin Dermatol. 2000; 18, 499-511).

Therefore, it would be desirable to have a composition capable of reinforcing the mucosa, increasing its barrier effect, decrease its permeability to reduce or prevent penetration, among others, of viruses, such as SARS-CoV-2, contaminants, such as biocides.

## DESCRIPTION OF THE INVENTION

A first aspect of the present invention relates to a composition comprising:

between 5% and 20% by weight with respect to the total weight of the composition of a lipophilic base selected from isopropyl myristate (MI), octyl palmitate, oleyl oleate, ethyl oleate, lauryl lactate, cetostearyl stearate, pentaerythrityl dioleate, isopropyl stearate, isopropyl linoleate, isodecyl oleate, octyl dodecyl myristate, isostearyl isostearate, propylene glycol, glycerol and/or a mixture of medium chain fatty acids (MCTG) selected from caprylic acid and capric acid; and
between 80% and 95% by weight with respect to the total weight of the composition of petroleum jelly or paraffin.

In another embodiment, the invention relates to the composition defined above, wherein the lipophilic base is isopropyl myristate (MI), preferably in an amount of between 5% and 10%.

In another embodiment, the invention relates to the composition defined above, wherein the lipophilic base is selected from propylene glycol and a mixture of medium chain triglycerides (MCTG) selected from glycerol, caprylic acid and capric acid, and preferably wherein the propylene glycol is in an amount of between 5% and 10% by weight with respect to the total weight of the composition and the mixture of medium chain triglycerides (MCTG) is in an amount of between 5% and 20% by weight with respect to the total weight of the composition.

In another embodiment, the invention relates to the composition defined above, wherein the petroleum jelly is white petroleum jelly.

In another embodiment, the invention relates to the composition defined above, further comprising between 10% and 50% by weight with respect to the total weight of the composition of one or more oils, preferably wherein the oils are selected from olive oil, almond oil and/or peanut oil.

In another embodiment, the invention relates to the composition defined above, further comprising between 1% and 2% by weight with respect to the total weight of the composition of one or more antibacterial and antiviral components, preferably wherein the antibacterial and antiviral components are selected from tea tree oil, thyme, eucalyptus, clove, lavender, oregano, basil, rosemary, marjoram, anise, bay leaf, geranium, sage, ginger, mint, artemisia, Jamaica pepper, lemon and/or garlic.

In another embodiment, the invention relates to the composition defined above comprising:

10% by weight with respect to the total weight of the composition of isopropyl myristate; and
90% by weight with respect to the total weight of the composition of white petroleum jelly.

In another embodiment, the invention relates to the composition defined above comprising:

10% by weight with respect to the total weight of the composition of propylene glycol, 10% by weight with respect to the total weight of the composition of a mixture of medium chain triglycerides selected from glycerol, caprylic acid and capric acid; and
80 % by weight with respect to the total weight of the composition of white petroleum jelly.

Another aspect of the invention relates to a pharmaceutical composition comprising the composition defined above and one or more pharmaceutically acceptable excipients.

Excipients must be "acceptable" in the sense of being compatible with the other ingredients of the composition and of not being harmful to those applying said composition.

The compositions of the present invention may be administered in the form of any pharmaceutical formulation, whose nature, as is well known, will depend on the nature of the active ingredient and on its administration route. In principle, any administration route may be used, for example, oral, parenteral, nasal, ocular, rectal, and topical.

Another aspect of the invention relates to the composition defined above, for use thereof as a medical product.

Another aspect of the invention relates to a medical product comprising the composition defined above.

Another aspect of the invention relates to the use of the composition defined above, for manufacturing a medical product for preventing infection from a virus selected from influenza, adenovirus, rotavirus, and SARS-CoV-2, preferably for preventing infection from SARS-CoV-2 virus, and more preferably for preventing the COVID-19 disease.

Another aspect of the invention relates to the use of the composition defined above, for manufacturing a medical product.

Another aspect of the invention relates to the use of the composition defined above, for manufacturing a medical product for preventing infection from a virus selected from influenza, adenovirus, rotavirus, and SARS-CoV-2, preferably for preventing infection from SARS-CoV-2 virus, and more preferably for preventing the COVID-19 disease.

Another aspect of the present invention relates to a method for preventing a disease in a subject in need thereof, particularly in humans, which comprises administering to said subject an effective amount of the composition defined above.

Another aspect of the present invention relates to a method for preventing infection from a virus selected from influenza, adenovirus, rotavirus, and SARS-CoV-2, preferably for preventing infection from SARS-CoV-2 virus, and more preferably for preventing the COVID-19 disease, in a subject in need thereof, particularly in humans, which comprises administering to said subject an effective amount of the composition defined above.

Another aspect of the relates to the use of the composition defined above for preventing a virus or a contaminant from penetrating through the oral or nasal mucosae, preferably through the oral mucosa.

In another embodiment, the invention relates to the use of the composition defined above for preventing a virus from penetrating through the oral or nasal mucosae, preferably through the oral mucosa, preferably wherein the virus is selected from influenza, adenovirus, rotavirus, or SARS-CoV-2, and more preferably wherein the virus is SARS-CoV-2.

In another embodiment, the invention relates to the use of the composition defined above for preventing a contaminant from penetrating through the oral or nasal mucosae, preferably through the oral mucosa, preferably wherein the contaminant is a biocide.

Another aspect of the invention relates to a method for preventing a virus or a contaminant from penetrating through the oral or nasal mucosa, preferably through the oral mucosa, comprising the administration of the composition defined above.

In another embodiment, the invention relates to the prevention method defined above, wherein the virus is SARS-CoV-2.

In another embodiment, the invention relates to the prevention method defined above, wherein the contaminant is a biocide.

Another aspect of the invention relates to the use of the medical product comprising the composition defined above for preventing infection from a virus selected from influenza, adenovirus, rotavirus, and SARS-CoV-2, preferably for preventing infection from SARS-CoV-2 virus, and more preferably for preventing the COVID-19 disease.

Another aspect of the invention relates to a medical device comprising the composition defined above.

Throughout the description, the term "contaminant" refers to one or more chemical substances, such as biocides, herbicides, pesticides, urban waste, oil or ionizing radiation the effect of which could cause diseases in living beings and in ecosystems or the environment. 5

The term "biocide" refers to one or more substances or mixtures thereof that are composed of, or generate, one or more synthetic or natural active chemical substances or microorganisms the purpose of which is to destroy, counteract, neutralize, prevent the action or otherwise exert control over any harmful organism by any means other than mere physical or mechanical action.

As it is used herein, the term "prevention" refers to preventing the onset of the disease that occurs in a patient who is predisposed or has risk factors, but still has no symptoms of the disease. Prevention also includes preventing the recurrence of a disease in a subject who has previously suffered from said disease.

Throughout the description and the claims, the word "comprises" and its variants are not intended to exclude other technical features, additives, components or steps. For those skilled in the art, other objects, advantages and features of the invention may be partially deduced from both the description and the embodiment of the invention. The following examples are provided by way of illustration and are not intended to limit the present invention.

## EXAMPLES

Next, the invention will be illustrated by means of assays carried out by the inventors.

### Example 1: **Lipophilic-based composition**

**Composition F-MI.-** Mix by gently stirring isopropyl myristate with white petroleum jelly

- 10% isopropyl myristate; and
- 90 % white petroleum jelly,

wherein the percentages are expressed by weight with respect to the total weight of the composition.

**Composition F-MCTG.-** Add the propylene glycol and the mixture of medium chain triglycerides (MCTGs) to the white petroleum jelly, wherein the MCTG mixture is composed of glycerol, caprylic acid and capric acid. Mix until obtaining a homogeneous paste.

- 10% propylene glycol;
- 10% medium chain triglycerides; and
- 80% white petroleum jelly,

wherein the percentages are expressed by weight with respect to the total weight of the composition.

### Example 2: **Evaluation of the barrier function by means of transmucosal water loss**

Of the F-MI composition described in Example 1, the loss of transmucosal water in the buccal and sublingual mucosa was evaluated using a Tewameter TM300 on a Nuclepore synthetic membrane.

The use of both animal and human biological membranes is essential to increase knowledge concerning the skin barrier, or the oral and nasal mucosa. However, ethical reasons or the complex methods for their attainment, preservation, and reproducibility, as well as their high cost, make it necessary to find synthetic membranes with a behavior similar to that of biological ones, therefore eliminating the limitations of the previous ones. Furthermore, the use of artificial membranes will obviate the great intra- and inter-individual variability.

Based on the above, two types of membranes, i.e., artificial and biological, have been used in order to verify the similarity or differences between them. Specifically, the following membranes are used:

- Whatman® Nuclepore™ artificial membranes, made of polycarbonate and with a pore size of 0.05 $\mu$m, which have been shown to have a permeability similar to that of human mucosae.

- porcine sublingual mucosae. Pig tongues were obtained from the Faculty of Pharmacy of the University of Barcelona, from the Hospital de Bellvitge campus with the protocols of the ethics committee and the supervision of said facility. Samples were dermatomized to a thickness of 500-700 $\mu$m (Dermatome GA630, Aesculap) and portions of the sublingual oral mucosa were obtained such that they fit into the Franz diffusion cells. Furthermore, in order to know the specific thickness, each portion of mucosa was measured with a digital micrometer (40X MAHR).

For the study of the barrier function of the mucosae, transmucosal water loss by means of Tewameter TM300 has initially been used in both Nuclepore synthetic membrane and sublingual oral mucosa.

Measurements of transepidermal water loss (TEWL) are carried out in Franz static diffusion cells (FDC) (3 ml, 1.86 $cm^2$, Lara-Spiral). These consist of a donor chamber and a receiver chamber (3 ml in volume) which are separated by a membrane for example, skin, mucosa, or artificial membrane. The receiver compartment of the lower chamber contains the receiver fluid containing 1% bovine serum albumin (Sigma Aldrich) and 0.04% gentamicin sulfate (Sigma Aldrich) in phosphate-buffered saline (pH = 7.6).

It is filled with a syringe with a needle and making sure that there are no bubbles in the liquid or at the lower part of the membrane. The cells are placed in a thermostatic bath (Julabo) for acclimatization until reaching a temperature of 32 $\pm$ 1°C.

Once the cells have stabilized for 1 hour and have reached the optimum temperature, TEWL measurements can be performed with a Tewameter®. The device is placed in the opening of the FDC, therefore, it gives a continuous response of the transepidermal water loss values. Furthermore, it contains two vertically located sensors that indicate two values of temperature and relative humidity (% RH), respectively. The measurements are performed before the application, 70 $\mu$l of composition are deposited and re-evaluated 1 hour after application. Furthermore, a control measure must be performed (without any type of application), measurements are performed in duplicate.

Table 1 describes the results of the composition F-MI of the invention with which TEWL is also evaluated with porcine sublingual mucosa (Table 1).

**Table 1.** Transepidermal water loss from Nuclepore synthetic membrane, from porcine sublingual mucosa, and from the same mucosae modified by applying the different compositions

| Compositions | TEWL 1 h Nuclepore (g/hm$^2$) | TEWL 1 h Sublingual mucosa (g/hm$^2$) |
|---|---|---|
| Nuclepore control | 80.8 | -- |
| Sublingual mucosa control | -- | 72.4 |
| Composition F-MI Lipophilic base MI | 2.6 | 6.5 |
| Composition F-MCTG | 3.6 | 3.0 |

The significant permeability of both the artificial membrane 80 g/hm$^2$ and sublingual mucosa 72 g/hm$^2$ with respect to the permeability of the skin, which is usually between approximately 5 and 10 g/hm$^2$, should be highlighted. The compositions of the invention greatly reduce water permeability (over 90%) both through the synthetic membrane and through the mucosa.

**Example 3: Kinetic drug permeation using vertical Franz diffusion cells**

Four drugs are evaluated to study the permeation characteristics of the treated and untreated membranes to the passage of active ingredients. Furthermore, porcine skin permeation is studied. As mentioned in the introduction, the barrier structure of the skin differs greatly from that of the mucosae. In this sense, it will be evaluated whether the mucosae modified both by lipophilic compositions and by compositions that mimic the stratum corneum could have a barrier effect similar to that of the skin.

Drugs were selected based on their different solubility and permeability characteristics. These two factors are directly related to the absorption process. Each of them belonging to a Biopharmaceutical Classification System (BCS) group. Additionally, this selection was made based on their pharmacological characteristics and frequent use as complementary therapy in the treatment of COVID.

The evaluated drugs were caffeine (CAF), ibuprofen (IBU), dexamethasone (DEX), and ivermectin (IVE) dissolved in methanol at a concentration of 1% for each active ingredient. Next, the main physicochemical properties which are important in permeability through keratin tissues, such as the skin and mucosae, and the classification to which they belong are described in detail. One drug from each class has been chosen:

Class I: high solubility (hydrophilic)-high permeability, caffeine
Class II: low solubility (hydrophobic)-high permeability, ibuprofen
Class III: high solubility (hydrophilic) - low permeability, dexamethasone
Class IV: low solubility (hydrophobic)-low permeability, ivermectin

Table 2. Compounds to be evaluated, physicochemical properties, and classification.

| Compounds | LogP (pH 7.4) | MW | Group |
|---|---|---|---|
| Caffeine (CAF) | -0.07 | 194.2 | 1 |
| Ibuprofen (IBU) | 3.97 | 206.3 | 2 |
| Dexamethasone (DEX) | 1.74 | 392.5 | 3 |
| Ivermectin (IVE) | 5.83 | 875.1 | 4 |

As in the previous TEWL study, sublingual mucosa and porcine skin dermatomized to a thickness of 500-700 $\mu$m are used. Kinetic diffusion studies were performed using a vertical diffusion cell (Lara Spiral, Couternon, France (1.86 cm$^2$ and 3 ml). The receiver fluid (RF) used was PBS:EtOH (1:1). A water bath was set at 43°C to obtain a membrane surface temperature of $32 \pm 1$°C.

Parameters such as TEWL, humidity, and temperature for the skin and mucosae were determined before the start of the test with Tewameter TM 300 (Courage + Khazaka, Cologne, Germany). 70 $\mu$l of composition F-MI is deposited in each mucosa. After 1 h, the TEWL is measured again (Table 3).

Table 3. Application of the composition F-MI and evaluation of transepidermal water loss, from the skin, from porcine sublingual mucosa and from the same mucosa modified by applying composition F-MI before applying the drugs.

| Sample | Amount applied | TEWL (g/m$^2$/h) |
|---|---|---|
| Skin | --- | 13.68$\pm$1.22 |
| Sublingual mucosa | --- | 84.72$\pm$4.44 |
| F-MI.* Sublingual mucosa + F-MI Lipophilic base MI | 51.87 mg | 4.17$\pm$1.73 |
| * Numbered composition according to Table 1 | | |

These TEWL results of the modified mucosae corroborate those obtained previously expressed in Table 1.

Subsequently, the drugs are deposited to determine their kinetics. To that end, 300 $\mu$l (infinite dose) of the 1% solution of the 4 drugs in methanol were applied to each Franz cell, in triplicate. Aliquots of 0.2 ml were collected at different times (30 min, 1 h, 2 h, 4 h). The active ingredients were diluted in suitable graduated flasks and filtered through a 0.22 $\mu$m nylon filter (Cameo, Sigma-Aldrich, St Louis, U. S.A.). They were then further analyzed with a high performance liquid chromatography diode array detector (HPLC-DAD).

All analyses were performed with reverse phase HPLC, using HPLC Agilent 1620 Infinity II LC System (Waldbronn, Germany) equipped with a quaternary pump (G7111B), autoinjector (G7167A), multi-column thermostat (G7116A), and WR diode-array detector (G7115A). The software used was OpenLab. The validation of the analytical procedures followed the guidelines developed by the International Conference on Harmonization (ICH) (Ich, ICH Topic Q2 (R1) Validation of Analytical Procedures: Text and Methodology. Int. Conf. Harmon., 2005; 1994, (November 1996), 1-17). The ICH guidelines were followed to obtain the calibration curve, the limit of quantification (LoQ), and the limit of detection (LoD). The HPLC-DAD analytical conditions and the method for the four active ingredients are described in detail in Table 4.

**Table 4.** Conditions of the method which are used in HPLC/DAD analysis for caffeine, ibuprofen, dexamethasone, and ivermectin.

| | Caffeine | Ibuprofen | Dexamethasone | Ivermectin |
|---|---|---|---|---|
| **Extractor solvent** | LiChrosolv® methanol | Methanol | Methanol | Methanol |
| **Column** | Lichrosphere® 100RP-18, 5 $\mu$m | Lichrosphere® 100RP-18, 5 $\mu$m | Lichosphere® 100RP-18, 5 $\mu$m | Lichrosphere® 100RP-18, 5 $\mu$m |
| **Wavelength (nm)** | 280 | 221 | 240 | 240 |
| **Injection volume ($\mu$l)** | 40 | 40 | 40 | 40 |
| **Mobile phase** | Methanol/Water 60:40-90:10 (15') - 90:10(15') - 60:40 (10') | Methanol/Water 60:40-90:10 (15') - 90:10(15') - 60:40 (10') | Methanol/Water 60:40-90:10 (15') - 90:10(15') -60:40 (10') | Methanol/Water 60:40-90:10 (15') -90:10(15') - 60:40 (10') |
| **Flow (ml/min)** | 1 | 1 | 1 | 1 |
| **Linear regression ($R^2$) equation** | $A$ = 51,512 [$CAF$] + 2,002 (0,9993) | $A$ = 58,407[$IBU$] - 8,753 (0.9996) | A= 51,352[DS] + 2,745 (0.9999) | $A$ = 42,000[$IVER$] -4,005 (0.9996) |
| **LoD/LoQ ($\mu$g/ml)** | 0.82/2.49 | 0.50 / 1.52 | 0.23/0.70 | 0.55/1.66 |

The release of the active pharmaceutical ingredient (API) was evaluated through the released cumulative amount (Qn, $\mu$g/cm$^2$), which corresponds to the cumulative amount of API quantified in the receiver liquid per surface area of the sample (Thakker, K.D.; Chern, W.H. Development and validation of in vitro release tests for semisolid dosage forms-case study. Dissolution Technol. 2003, 10, 10-15). The equation is as follows (1):

$$(1) \qquad Qn = \frac{Cn \times Vc + \sum_{i=1}^{n-1}(Ci \times Vs)}{A}$$

Wherein: Qn is the cumulative amount of active ingredient released at time n ($\mu$g/cm$^2$); Cn is the concentration of active ingredient in the sample ($\mu$g/ml); Vc is the volume of the vertical diffusion cell (7 ml); $\sum_{i=1}^{n-1} Ci$ is the sum of the API concentrations ($\mu$g/ml) determined at sampling intervals 1 to n-1; Vs is the volume of the sample, and A is the surface area of the sample (1.77 cm$^2$).

Two parameters were used, Qn and % API release, to obtain graphs showing absorption and penetration kinetics (Y-axis). Time or its square root ($\sqrt{t}$) is indicated on the X-axis. The percentage of drug released over time best fit the equation, which represents the absorption kinetics, as described by Mallandrich *et. al.* (Mallandrich, M.; Fernández-Campos, F.; Clares, B.; Halbaut, L.; Alonso, C.; Coderch, L.; Garduño-Ramírez, M.L.; Andrade, B.; Del Pozo, A.; Lane, M.E.; et al. Developing Transdermal Applications of Ketorolac Tromethamine Entrapped in Stimuli Sensitive Block Copolymer Hydrogels. Pharm. Res. 2017, 34, 1728-1740). This step allowed determining the best absorption model to represent the penetration kinetics of the API through the different membranes. The model was obtained with the non-linear regression software STATGRAPHICS plus 5 (Statgraphics Technologies, Inc., Virginia, U. S.A.), and the best equation was selected based on the highest correlation coefficient corrected by the number of degrees of freedom (R2 DoF). Once having defined the model, it was possible to calculate other parameters, such as the flow (J), permeability coefficient (Kp), delay time (TI), maximum concentration (Cmax), maximum time (tmax), and area under the curve (AUC). All results are expressed as mean $\pm$ standard deviation (SD).

### 3.1 Caffeine permeation

The kinetic permeation assay was performed in triplicate for caffeine on porcine skin, mucosa, and modified mucosa after depositing composition F-MI. The release of the active pharmaceutical ingredient was evaluated through the released cumulative amount (Qn, $\mu$g/cm$^2$), which is equivalent to the total amount of API quantified in the receiver liquid per unit area. Other kinetic parameters were determined (flux, Cmax) as described in detail in the experimental section. The percentage of drug released over time was measured for the different four active ingredients, obtaining permeation properties of each one. The results are shown in Table 5.

**Table 5**. Mean values of area under the curve (AUC), flow (J), permeability coefficient (Kp), latency time (TL) and maximum concentration (Cmax) for caffeine through the skin, sublingual mucosa and sublingual mucosae with F-MI.

| Parameter | SKIN CAF | MUCOSA CAF | MUCOSA F-MI CAF |
|---|---|---|---|
| Area under the curve, AUC (mg/cm$^2$) | 0.41$\pm$0.27 | 2.19$\pm$0.93 | 0.49$\pm$0.13 |
| Flow, J, ($\mu$g/cm$^2$/h) | 75.09$\pm$51.32 | 428,40$\pm$81,41 | 102.73$\pm$30.73 |
| Permea. coeff., Kp ($10^{-3}$cm/h) | 6.06$\pm$4.12 | 34.55$\pm$6.56 | 8.28$\pm$2.48 |
| Latency time, LT (h) | 0.65$\pm$0.24 | 0.04$\pm$0.34 | 0.10$\pm$0.08 |
| Conc. Max., Cmax ($\mu$g/ml) | 107.23$\pm$57.7 3 | 514.46$\pm$119.97 | 118.25$\pm$34.84 |

The application of composition F-MI gives the mucosa an impermeability that is very similar to that of the skin. Therefore, for a low molecular weight hydrophilic compound indicating a high permeability such as caffeine, it exhibits an impermeability similar to that of the skin when composition F-MI is applied to the mucosae.

### 3.2 Ibuprofen permeation

The kinetic permeation assay was performed in triplicate for ibuprofen on porcine skin, mucosa, and modified mucosa after depositing composition F-MI. The release of the active pharmaceutical ingredient was evaluated through the released cumulative amount (Qn, $\mu$g/cm$^2$), which is equivalent to the total amount of API quantified in the receiver liquid per unit area. Other kinetic parameters were determined (flux, Cmax) as described in detail in the experimental section. The percentage of drug released over time was measured for the different four active ingredients, obtaining permeation properties of each one. The results are shown in Table 6.

**Table 6.** Mean values of area under the curve (AUC) flow (J), permeability coefficient (Kp), latency time (TL) and maximum concentration (Cmax) for ibuprofen through the skin, sublingual mucosa and sublingual mucosae with F-MI.

| Parameter | SKIN IBU | MUCOSA IBU | MUCOSA F-MI IBU |
|---|---|---|---|
| Area under the curve, AUC (mg/cm$^2$) | 0.10 $\pm$ 0.01 | 1.27$\pm$0.49 | 1.32$\pm$0.16 |
| Flow, J, ($\mu$g/cm$^2$/h) | 24.79 $\pm$ 2.75 | 346.91$\pm$5.87 | 498.25$\pm$49. 45 |
| Permea. coeff., Kp ($10^{-3}$cm/h) | 2.05 $\pm$ 0.23 | 28.67$\pm$0.49 | 41.18$\pm$4.09 |
| Latency time, LT (h) | -0.24 $\pm$ 0.19 | 0.17$\pm$0.07 | 0.23$\pm$0.01 |
| Conc. Max., Cmax (pg/ml) | 21.93 $\pm$ 1.98 | 340.76 $\pm$ 63.62 | 406.04$\pm$36. 48 |

### 3.3. Dexamethasone permeation

The kinetic permeation assay was performed in triplicate for dexamethasone on porcine skin, mucosa, and modified mucosa after depositing composition F-MI. The release of the active pharmaceutical ingredient was evaluated through the released cumulative amount (Qn, $\mu$g/cm$^2$), which is equivalent to the total amount of API quantified in the receiver liquid per unit area. Other kinetic parameters were determined (flux, Cmax) as described in detail in the experimental section. The percentage of drug released over time was measured for the different four active ingredients, obtaining permeation properties of each one. The results are shown in Table 7.

**Table 7.** Mean values of area under the curve (AUC) flow (J), permeability coefficient (Kp), latency time (LT) and maximum concentration (Cmax) for dexamethasone through the skin, sublingual mucosa and sublingual mucosae with F-MI.

| Parameter | SKIN DEX | DEX MUCOSA | MUCOSA F-MI DEX |
|---|---|---|---|
| Area under the curve, AUC (mg/cm$^2$) | 0.03$\pm$0.01 | 0,83$\pm$0,19 | 1.10$\pm$0.18 |

(continued)

| Parameter | SKIN DEX | DEX MUCOSA | MUCOSA F-MI DEX |
|---|---|---|---|
| Flow, J, ($\mu$g/cm$^2$/h) | 5,84$\pm$3,46 | 335,25$\pm$61,25 | 330.43$\pm$11.68 |
| Permea. coeff., Kp (10$^{-3}$cm/h) | 0,49$\pm$0,29 | 28,17$\pm$5,15 | 27.77$\pm$0.98 |
| Latency time, LT (h) | 0,62$\pm$0,16 | 0,31$\pm$0,13 | 0.36$\pm$0.04 |
| Cone. Max., Cmax ($\mu$g/ml) | 67,41$\pm$95,05 | 315,34$\pm$87,38 | 363.51$\pm$41.15 |

### 3.4. Ivermectin permeation

The kinetic permeation assay was performed in triplicate for ivermectin on porcine skin, mucosa, and modified mucosa after depositing composition F-MI. The release of the active pharmaceutical ingredient was evaluated through the released cumulative amount (Qn, $\mu$g/cm$^2$), which is equivalent to the total amount of API quantified in the receiver liquid per unit area. Other kinetic parameters were determined (flux, Cmax) as described in detail in the experimental section. The percentage of drug released over time was measured for the different four active ingredients, obtaining permeation properties of each one. The results are shown in Table 8.

**Table 8.** Mean values of area under the curve (AUC) flow (J), permeability coefficient (Kp), latency time (LT) and maximum concentration (Cmax) for ivermectin through the skin, sublingual mucosa and sublingual mucosae with F-MI.

| Parameter | SKIN IVER | IVER MUCOSA | MUCOSA F-MI IVER |
|---|---|---|---|
| Area under the curve, AUC (mg/cm$^2$) | 0.026$\pm$0.002 | 0.13$\pm$0.02 | 0.17$\pm$0.01 |
| Flow, J, ($\mu$g/cm$^2$/h) | 3.13$\pm$0.86 | 63.75$\pm$1.57 | 87.23$\pm$21.8 3 |
| Permea. coeff., Kp (10$^{-3}$cm/h) | 0.25$\pm$0.07 | 5.06$\pm$0.13 | 6.92$\pm$1.73 |
| Latency time, LT (h) | 0.59$\pm$0.28 | 0,24$\pm$0,0006 4 | 0.28$\pm$0.02 |
| Conc. Max., Cmax (pg/ml) | 7.38$\pm$0.13 | 44.45$\pm$1.68 | 69.66$\pm$18.6 1 |

Ivermectin, as expected, is the active ingredient with the lowest permeability both in the skin and in the mucosa. Furthermore, the difference between the skin and mucosa is not as pronounced as in the case of dexamethasone. In this case, the permeability of ivermectin through the unmodified mucosa is 20 times higher compared to the skin.

### Example 4: Kinetic permeation of biocides using vertical Franz diffusion cells

Three biocides are also evaluated to study the permeation characteristics of the treated and untreated membranes to the passage of active ingredients. Furthermore, porcine skin permeation is studied. As mentioned in the introduction, the barrier structure of the skin differs greatly from that of the mucosae. In this sense, whether the mucosae modified by the compositions of the invention could have a barrier effect similar to that of the skin will be evaluated.

The biocides evaluated were fungitrol (FUN), propiconazole (PRO), and permethrin (PER) dissolved in ethanol at a concentration of 1% for each active ingredient. In this case, the size is very similar between 300 and 400 MW, and the hydrophilicity would be similar for fungitrol and propiconazole, with permethrin being the most hydrophobic active ingredient.

**Table 9.** Biocides and their physicochemical properties.

| Biocides | LogP (pH 7.4) | MW | Chemical structure | Reference |
|---|---|---|---|---|
| Fungitrol (FUN) | 2.4 | 281.1 | $C_8H_{12}INO_2$ | National Center for Biotechnology Information. PubChem Compound Summary for CID 62097, 3-lodo-2-propynyl butylcarbamate. |
| Propiconazole (PRO) | 3.7 | 342.2 | $C_{15}H_{17}Cl_2N_3O_2$ | National Center for Biotechnology Information. PubChem Compound Summary for CID 43234, Propiconazole. |

(continued)

| Biocides | LogP (pH 7.4) | MW | Chemical structure | Reference |
|---|---|---|---|---|
| Permethrin (PER) | 6.5 | 391.3 | $C_{21}C_{l2}H_{20}O_3$ | National Center for Biotechnology Information. PubChem Compound Summary for CID 40326, Permethrin. |

**Table 10.** Conditions of the method which are used in HPLC/DAD analysis for fungitrol, propiconazole, and permethrin

| | **Fungitrol** | **Propiconazole** | **Permethrin** |
|---|---|---|---|
| **Solvent Extractor** | LiChrosolv® methanol | LiChrosolv® methanol | LiChrosolv® methanol |
| **Column** | Zorbax Eclipse XDB C18 (4.6x150mm, 5 $\mu$m) | Zorbax Eclipse XDB C18 (4.6x150mm, 5 $\mu$m) | Zorbax Eclipse XDB C18 (4.6x150mm, 5 $\mu$m) |
| **Wavelength (nm)** | 200 | 210 | 210 |
| **Injection volume ($\mu$l)** | 20 | 20 | 20 |
| **Mobile phase** | Acetonitrile/water 52/48 - 85/15 (10') 85/15 (8') 52/48 (5') | Acetonitrile/water 52/48 - 85/15 (10') 85/15 (8') 52/48 (5') | Acetonitrile/water 52/48 - 85/15 (10') 85/15 (8') 52/48 (5') |
| **Flow (ml/min)** | 1 | 1 | 1 |
| **Linear regression ($R^2$) equation** | y = 7.8218x + 0.6291 $R^2$ = 0.9986 | y = 41,302x - 0,7125 $R^2$ = 0,9987 | y = 34.733x + 0.0206 $R^2$ = 0.9986 |
| **LoD/LoQ ($\mu$g/ml)** | 1.09 / 3.31 | 0.37 / 1.11 | 0.49 / 1.49 |

### 4.1. Fungitrol permeation

The kinetic permeation assay was performed in triplicate for fungitrol on porcine skin, mucosa, and modified mucosa after depositing composition F-MI. The release of the active pharmaceutical ingredient was evaluated through the released cumulative amount (Qn, $\mu$g/cm$^2$), which is equivalent to the total amount of API quantified in the receiver liquid per unit area. Other kinetic parameters were determined (flux, Cmax) as described in detail in the experimental section. The percentage of drug released over time was measured for the different four active ingredients, obtaining permeation properties of each one. The results are shown in Table 11.

**Table 11.** Mean values of area under the curve (AUC) flow (J), permeability coefficient (Kp), latency time (TL) and maximum concentration (Cmax) for Fungitrol through the skin, sublingual mucosa and sublingual mucosae with F-MI.

| Parameter | SKIN FUN | MUCOSA FUN | MUCOSA F-MI FUN |
|---|---|---|---|
| Area under the curve, AUC (mg/cm$^2$) | 0.024±0.016 | 0.696±0.699 | 0.020±0.00 2 |
| Flow, J, ($\mu$g/cm$^2$/h) | 6.02±3.93 | 62.88±29.97 | 5.59±1.32 |
| Permea. coeff., Kp (10$^{-3}$cm/h) | 1.00±0.40 | 6.80±3.26 | 0.61±0.14 |
| Latency time, LT (h) | 0.70±0.00 | -0.34±1.32 | 0.65±0.08 |
| Conc. Max., Cmax (pg/ml) | 14.94±9.75 | 168.10±96.2 0 | 12.61±1.51 |

The application of F-MI confers to the mucosa an impermeability that is very similar to that of the skin. Therefore, for a hydrophobic compound of intermediate molecular weight, it exhibits a permeability similar to that of dexamethasone in skin, and to ivermectin in the mucosa.

**4.2. Propiconazole permeation**

The kinetic permeation assay was performed in triplicate for propiconazole on porcine skin, mucosa, and modified mucosa after depositing composition F-MI. The release of the active pharmaceutical ingredient was evaluated through the released cumulative amount (Qn, $\mu$g/cm$^2$), which is equivalent to the total amount of API quantified in the receiver liquid per unit area. Other kinetic parameters were determined (flux, Cmax) as described in detail in the experimental section. The percentage of drug released over time was measured for the different four active ingredients, obtaining permeation properties of each one. The results are shown in Table 12.

**Table 12.** Mean values of area under the curve (AUC) flow (J), permeability coefficient (Kp), latency time (LT) and maximum concentration (Cmax) for Propiconazole through the skin, sublingual mucosa and sublingual mucosae with F-MI.

| Parameter | SKIN PRO | MUCOSA PRO | MUCOSA F-MI PRO |
|---|---|---|---|
| Area under the curve, AUC (mg/cm$^2$) | 0.011±0.007 | 0.725±0.850 | 0.028±0.01 7 |
| Flow, J, ($\mu$g/cm$^2$/h) | 2.26±1.48 | 63.92±44.92 | 6.62±3.74 |
| Permea. coeff., Kp (10$^{-3}$cm/h) | 0.24±0.16 | 6.80±4.78 | 0.70±0.39 |
| Latency time, LT (h) | 0.60±0.015 | -0.04±1.68 | 1.66±1.00 |
| Conc. Max., Cmax (pg/ml) | 5.53±3.52 | 159.58±143. 25 | 12.32±9.89 |

The application of F-MI confers to the mucosa an impermeability that is very similar to that of the skin. Therefore, for a hydrophobic compound of intermediate molecular weight, it exhibits a permeability similar to that of ivermectin in skin and mucosa.

**4.3. Permethrin permeation**

The kinetic permeation assay was performed in triplicate for permethrin on porcine skin, mucosa, and modified mucosa after depositing composition F-MI. The release of the active pharmaceutical ingredient was evaluated through the released cumulative amount (Qn, $\mu$g/cm$^2$), which is equivalent to the total amount of API quantified in the receiver liquid per unit area. Other kinetic parameters were determined (flux, Cmax) as described in detail in the experimental section. The percentage of drug released over time was measured for the different four active ingredients, obtaining permeation properties of each one. The results are shown in Table 13.

**Table 13.** Mean values of area under the curve (AUC) flow (J), permeability coefficient (Kp), latency time (TL) and maximum concentration (Cmax) for Permethrin through the skin, sublingual mucosa and sublingual mucosae with F-MI.

| Parameter | SKIN PER | MUCOSA PER | MUCOSA F-MI PER |
|---|---|---|---|
| Area under the curve, AUC (mg/cm$^2$) | 0.039±0.004 | 0.74±0.79 | 0.063±83.18 |
| Flow, J, ($\mu$g/cm$^2$/h) | 2.93±0.23 | 58.67±45.51 | 3.85±4.00 |
| Permea. coeff., Kp (10$^{-3}$cm/h) | 0.20±0.015 | 4.02±3.12 | 0.26±0.27 |
| Latency time, LT (h) | -1.49±0.66 | -0.53±1.14 | -0.28±1.77 |
| Conc. Max., Cmax (pg/ml) | 9.35±0.11 | 157.66±135.4 9 | 15.70±20.27 |

The application of F-MI confers to the mucosa an impermeability that is very similar to that of the skin. Therefore, for a hydrophobic compound of intermediate molecular weight, it exhibits a permeability similar to that of ivermectin in skin and mucosa.

**Example 5: Kinetic permeation of a SARS-CoV-2 virus model using vertical Franz diffusion cells**

**5.1 Synthesis of the virus model**

In order to analyze the potential impermeabilization effect against SARS-CoV-2 exerted on the mucosa by the compositions of the invention, a chemical model of the virus was required. For its design, fundamental aspects that characterize it such as its spherical morphology, its approximate size of 120 nm in diameter, and the cationic and hydrophobic nature of its surface, were taken into account.

Metallic nanoparticles coated on their surface with a bilayer of cationic gemini surfactants have been used. Specifically, gold nanoparticles have been chosen, since they have a characteristic strong absorption band in the visible part of the spectrum that can be easily characterized by UV-vis absorption spectroscopy, from which the concentration of particles, their size and polydispersity can be calculated. The amphiphilic compound 1,3-bis[(3-octadecyl-1-imidazolium) methyl]benzene 1.2Br was chosen as a coating surfactant, with the capacity to stabilize the gold surface, forming a monolayer around the gold core in which the positive charges derived from the imidazolium salts are close to the metallic surface and the hydrophobic chains (of 18 carbon atoms) are arranged towards the outside of the nanoparticle. This amphiphilic compound is also capable of assembling forming bilayers around the gold core, resulting in a cationic surface of the nanoparticle, and generating the virus model.

For the synthesis of these nanoparticles, a procedure was followed that is briefly summarized below. Commercial citrate-stabilized gold nanoparticles with a concentration of ~3.8 $10^9$ particles/ml and diameter of 100 nm were used. These nanoparticles were functionalized with 1.2Br by treatment with a 0.55 mM aqueous solution of the amphiphilic compound, using sonication followed by stirring at room temperature for 12 hours. The obtained nanoparticles were characterized by UV-vis absorption spectroscopy and Dynamic Light Scattering (DLS). This allowed knowing their concentration ($6.4 \cdot 10^{-12}$ M), as well as their size, which corresponds to a diameter of 120 nm (PDI 0.15) and its Z potential is ca. 45 mV, which indicates its cationic nature and its high stability.

**5.2 Virus model permeation**

Lastly, another technological challenge has been the production of new artificial virus models that make it possible to evaluate all those compositions with the potential to protect mucosae from SARS-CoV-2 or other contaminants. For this, the viability of the gold nanoparticles previously described and synthesized as a model of the SARS-CoV-2 virus was studied, both with the artificial membrane and with biological membranes.

The experiment was performed with the same design as the drug permeation assay. Nuclepore membrane, buccal mucosa and sublingual mucosa were used as permeation membrane. The biological membranes were surgical surpluses from the facility of the Faculty of Medicine, University of Barcelona, Bellvitge campus.

The handling of the mucosa is carried out following the basic safety standards for protection against possible exposure to pathogenic viral agents. The material used (scissors, scalpel, etc.) is sterilized in the steribox at 150°C for 20 minutes while the biological material remains are disposed of in special containers that are kept at low temperatures. The oral mucosa is cut into strips of about 3-4 cm and 500 $\mu$m thick using an electric dermatome. This thickness makes it possible to represent the entire mucosal barrier and, therefore, to study drug passage through loose connective tissue, the lining epithelium, the basal cell layer and the prickle cell layer. The mucosa is placed with the underside in contact with the fluid of the receiver compartment.

To perform the test, the receiver compartment is filled with water and 100 mg of protective composition F-MI are placed in the donor compartment. Initially, the F-MI was chosen because it is the most impermeable for the passage of water vapor and because it is the most impermeable with respect to the biocides evaluated. Next, 0.4 $\mu$l microliters of dispersion of the virus model are seeded. Said dispersion has a concentration of 0.595 $\mu$g/ml. Subsequently, the upper part of the cell is covered with Parafilm®. The temperature is maintained inside the cell at 37 $\pm$ 0.5°C. The sample is taken manually through the side tube of the Franz cell, at preset intervals every hour for 6 hours. The volume that has been removed is immediately added, with the replenishing medium.

After the permeation test is complete, the membrane was removed from the Franz cells and the excess composition was removed from the membrane surface. This was cleaned with gauze soaked in 0.05% sodium lauryl sulfate solution and washed 3 times with distilled water. The pieces of skin were placed in glass vials and the virus content was extracted with water. The supernatants were pipetted and analyzed, obtaining the amount of virus model retained in the mucosa. Extractions are placed in chromatography vials until their analysis by plasma mass spectrometry. Their determination is carried out according to the following methodology: in Teflon capsules (cleaned with Agua Regia), are tared and later, the sample is weighed and 0.5 ml of Aqua Regia is added. It is left overnight at 90°C. After cooling down, 10 ml of thiourea (200 ppm 1% HCl) are added.

Finally, it is covered, homogenized and weighed, in order to pass to glass tubes, washing the tubes with the etching solution. The analysis was performed by inductively coupled plasma mass spectrometry with the Agilent Technologies Inc. - 7500ce ICP-MS equipment.

Table 14 shows the retained virus model (amount present on the membrane after completion of the assay) and permeated virus model (amount present in the medium of the acceptor compartment of the cell) after its deposition on the different membranes.

The results of the modified membranes correspond to the values of the virus deposited on membranes previously treated with the composition F-MI with a lipophilic base of MI which gave a small value of TWEL. For this reason, it is a good candidate to be applied to mucous membranes and to study its protective nature against the virus model.

**Table 14.** Amount of retained and permeated model virus on membranes and membranes modified with an impermeabilization composition, expressed in $\mu$g/ml of gold.

| | Retained Amount | | Permeated Amount | |
|---|---|---|---|---|
| | Membrane $\mu$g/ml, $10^{-3}$ | F-MI modified membrane $\mu$g/ml $10^{-3}$ | Membrane $\mu$g/ml $10^{-3}$ | F-MI modified membrane $\mu$g/ml $10^{-3}$ |
| **Nucleopore** | 34.1$\pm$ | 23.8 | 305.7 | 3.2 |
| **Buccal mucosa** | 0.1 | --- | 232.2 | 4.8 |

In both results, retention and permeation, the barrier action of the impermeabilization composition is observed for all the membranes. This is especially relevant in the virus model permeation, with much lower values for the impermeable artificial and buccal membranes. This means great protection against the passage of the virus model.

Moreover, a great similarity is observed between the artificial membrane and buccal mucosa for the permeated virus. This similarity is maintained before and after the treatment of the membranes with the impermeabilization composition. Based on this, the artificial membrane could be used instead of the biological membrane for permeation tests.

With these results, it can be expected that the compositions of the invention protect people in general and a health worker in particular from being infected by a virus, such as, for example, SARS-CoV-2 and being protected from contaminants, such as, for example, biocides.

## Claims

1. A composition comprising:

   between 5% and 20% by weight with respect to the total weight of the composition of a lipophilic base selected from isopropyl myristate (MI), octyl palmitate, oleyl oleate, ethyl oleate, lauryl lactate, cetostearyl stearate, pentaerythrityl dioleate, isopropyl stearate, isopropyl linoleate, isodecyl oleate, octyl dodecyl myristate, isostearyl isostearate, propylene glycol, glycerol and/or a mixture of medium chain fatty acids (MCTG) selected from caprylic acid and capric acid; and
   between 80% and 95% by weight with respect to the total weight of the composition of petroleum jelly or paraffin.

2. The composition according to claim 1, wherein the lipophilic base is isopropyl myristate in an amount of between 5% and 10% by weight with respect to the total weight of the composition.

3. The composition according to claim 1, wherein the lipophilic base is selected from propylene glycol and a mixture of medium chain triglycerides (MCTG) selected from glycerol, caprylic acid and capric acid, wherein the propylene glycol is in an amount of between 5% and 10% by weight with respect to the total weight of the composition.

4. The composition according to any of claims 1 to 3, further comprising between 10% and 50% by weight with respect to the total weight of the composition of one or more oils.

5. The composition according to any of claims 1 to 4, further comprising between 1% and 2% by weight with respect to the total weight of the composition of one or more antibacterial and antiviral components.

6. The composition according to claim 1, comprising:

   10% by weight with respect to the total weight of the composition of isopropyl myristate; and
   90% by weight with respect to the total weight of the composition of white petroleum jelly.

7. A pharmaceutical composition comprising the composition according to any of claims 1 to 6 and one or more pharmaceutically acceptable excipients.

8. The composition according to any of claims 1 to 7, for use thereof as a medical product.

9. The composition according to any of claims 1 to 7, for use thereof in preventing infection from a virus.

10. The composition for use thereof according to claim 9, wherein the virus is selected from influenza, adenovirus, rotavirus, and SARS-CoV-2.

11. The composition according to any of claims 1 to 7, for the prevention of COVID-19 disease.

12. A method for preventing a virus or a contaminant from penetrating through the oral or nasal mucosae, comprising the administration of the composition according to any of claims 1 to 7.

13. The method according to claim 12, wherein the contaminant is a biocide.

14. A medical product comprising the composition according to any of claims 1 to 7.

15. A medical device comprising the composition according to any of claims 1 to 7.

Europäisches Patentamt

European Patent Office

Office européen des brevets

# EUROPEAN SEARCH REPORT

**Application Number**

EP 23 38 2737

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2021/249667 A1 (METRIOPHARM AG [CH]) 16 December 2021 (2021-12-16) * page 41 * | 1-15 | INV. A61K9/00 A61K9/06 A61K31/23 A61K47/10 A61K47/14 |
| Y | CN 114 802 849 A (LOU FEI) 29 July 2022 (2022-07-29) * claim 1; example 3 * | 1-15 | |
| Y | YAGHMOURI PEDRAM ET AL: "Petroleum jelly and COVID-19 prevention", BRATISLAVA MEDICAL JOURNAL, vol. 123, no. 06, 1 January 2022 (2022-01-01), pages 455-456, XP093116098, 18.01.2022 ISSN: 1336-0345, DOI: 10.4149/BLL_2022_071 Retrieved from the Internet: URL:http://www.elis.sk/download_file.php?product_id=7680&session_id=7h2tin52boct4emnh65101ntm2> * page 2, right-hand column * * abstract * | 1-15 | |
| Y | CN 1 416 887 A (HU ZIXIAO [CN]) 14 May 2003 (2003-05-14) * page 4, last paragraph; claim 1 * | 1-15 | TECHNICAL FIELDS SEARCHED (IPC) A61K |
| Y | RU 2 086 232 C1 (UFIM NII VAKTSIN I SYVOROTOK I [SU]) 10 August 1997 (1997-08-10) * page 3; claim 1 * | 1-15 | |
| X | WO 2004/100884 A2 (DMI BIOSCIENCES INC [US]) 25 November 2004 (2004-11-25) | 1 | |
| Y | * page 1, line 3 - line 4 * * page 16, line 19 - line 20 * | 1-15 | |

-/--

The present search report has been drawn up for all claims

| Place of search | Date of completion of the search | Examiner |
|---|---|---|
| Munich | 23 January 2024 | Neumann, Robert |

Europäisches
Patentamt
European
Patent Office
Office européen
des brevets

# EUROPEAN SEARCH REPORT

Application Number

EP 23 38 2737

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2006/098353 A1 (KYOWA HAKKO KOGYO KK [JP]; SUGISHITA JUN ET AL.) 21 September 2006 (2006-09-21) | 1 | |
| Y | * examples 7, 19, 20 * | 1-15 | |
| Y | Flexikon Basiscreme Dac -Doccheck: "02/01/2024, 15:05", , 7 January 2018 (2018-01-07), pages 1-4, XP093115405, Retrieved from the Internet: URL:https://flexikon.doccheck.com/de/Basis creme_DAC [retrieved on 2024-01-02] * the whole document * | 1-15 | |
| T | ALONSO CRISTINA ET AL: "A Synthetic Model of the Mucosa for Oral Penetration Studies", MEMBRANES, vol. 13, no. 12, 1 December 2023 (2023-12-01), page 905, XP093115150, CH ISSN: 2077-0375, DOI: 10.3390/membranes13120905 * the whole document * | | |

TECHNICAL FIELDS
SEARCHED    (IPC)

The present search report has been drawn up for all claims

| Place of search | Date of completion of the search | Examiner |
|---|---|---|
| Munich | 23 January 2024 | Neumann, Robert |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 23 38 2737

This annex lists the patent family members relating to the patent documents cited in the above-mentioned European search report.
The members are as contained in the European Patent Office EDP file on
The European Patent Office is in no way liable for these particulars which are merely given for the purpose of information.

23-01-2024

| Patent document cited in search report | | Publication date | Patent family member(s) | | Publication date |
|---|---|---|---|---|---|
| WO 2021249667 | A1 | 16-12-2021 | AU 2021288757 | A1 | 02-02-2023 |
| | | | BR 112022025134 | A2 | 27-12-2022 |
| | | | CA 3182022 | A1 | 16-12-2021 |
| | | | CN 116075306 | A | 05-05-2023 |
| | | | EP 4164651 | A1 | 19-04-2023 |
| | | | IL 298899 | A | 01-02-2023 |
| | | | JP 2023529022 | A | 06-07-2023 |
| | | | KR 20230027150 | A | 27-02-2023 |
| | | | US 2023218614 | A1 | 13-07-2023 |
| | | | WO 2021249667 | A1 | 16-12-2021 |
| CN 114802849 | A | 29-07-2022 | NONE | | |
| CN 1416887 | A | 14-05-2003 | NONE | | |
| RU 2086232 | C1 | 10-08-1997 | NONE | | |
| WO 2004100884 | A2 | 25-11-2004 | AU 2004238285 | A1 | 25-11-2004 |
| | | | BR PI0410505 | A | 20-06-2006 |
| | | | CA 2523000 | A1 | 25-11-2004 |
| | | | CN 1784212 | A | 07-06-2006 |
| | | | EP 1620066 | A2 | 01-02-2006 |
| | | | JP 2006528690 | A | 21-12-2006 |
| | | | KR 20060015588 | A | 17-02-2006 |
| | | | US 2005002876 | A1 | 06-01-2005 |
| | | | WO 2004100884 | A2 | 25-11-2004 |
| | | | ZA 200508952 | B | 25-04-2007 |
| WO 2006098353 | A1 | 21-09-2006 | CA 2601123 | A1 | 21-09-2006 |
| | | | JP WO2006098353 | A1 | 28-08-2008 |
| | | | US 2009012051 | A1 | 08-01-2009 |
| | | | WO 2006098353 | A1 | 21-09-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **S.H. WHITE** ; **D. MIREJOVSKY** ; **G.I. KING**. Structure of Lamellar Lipid Domains and Corneocyte Envelopes of Murine Stratum Corneum. An X-ray Diffraction Study. *Biochemistry*, 1988, vol. 27, 3725-3732
- **BOUWSTRA J** ; **PILGRAM G** ; **GOORIS G** ; **KOERTEN H** ; **PONEC M**. New aspects of the skin barrier organization. Skin Pharmacol. *Appl Skin Physiol.*, 2001, vol. 14 (1), 52-62
- **D. KESSNER** ; **A. RUETTINGER** ; **M.A. KISELEV** ; **S. WARTEWIG** ; **R.H.H. NEUBERT**. Properties of ceramides and their impact on the stratum corneum structure: A review - Part 2: Stratum corneum lipid model systems. *Skin Pharmacol. Physiol.*, 2008, vol. 21, 58-74
- **BESTE KINIKOGLU** ; **ODILE DAMOUR** ; **VASIF HASIRCI**. Tissue engineering of oral mucosa: a shared concept with skin. *J Artif Organs*, 2015, vol. 18, 8-19
- **AMIR H SHOJAEI**. Buccal Mucosa as A Route For Systemic Drug Delivery: A Review. *J. Pharm. Pharmaceut. Sci.*, 1988, vol. 1 (1), 15-30
- **WINNING TA** ; **TOWNSEND GC**. Oral mucosal embryology and histology. *Clin Dermatol.*, 2000, vol. 18, 499-511
- *Conference on Harmonization (ICH) (Ich, ICH Topic Q2 (R1) Validation of Analytical Procedures: Text and Methodology. Int. Conf. Harmon., 2005*, 1994, 1-17
- **THAKKER, K.D.** ; **CHERN, W.H.** Development and validation of in vitro release tests for semisolid dosage forms-case study. *Dissolution Technol.*, 2003, vol. 10, 10-15
- **MALLANDRICH, M.** ; **FERNÁNDEZ-CAMPOS, F.** ; **CLARES, B.** ; **HALBAUT, L.** ; **ALONSO, C.** ; **CODERCH, L.** ; **GARDUÑO-RAMÍREZ, M.L.** ; **AN-DRADE, B.** ; **DEL POZO, A.** ; **LANE, M.E. et al.** Developing Transdermal Applications of Ketorolac Tromethamine Entrapped in Stimuli Sensitive Block Copolymer Hydrogels. *Pharm. Res.*, 2017, vol. 34, 1728-1740